(19) Europäisches Patentamt
European Patent Office
Office européen des brevets

(11) Publication number: 0 227 160
B1

(12) **EUROPEAN PATENT SPECIFICATION**

(45) Date of publication of the patent specification: **11.04.90**

(21) Application number: **86202157.3**

(22) Date of filing: **03.12.86**

(51) Int. Cl.[4]: **C07C 51/14**, C07C 67/38, B01J 31/18, B01J 31/24, C07C 55/12, C07C 55/14, C07C 69/42, C07C 69/44

(54) **Process for the carbonylation of ethylenically unsaturated compounds.**

(30) Priority: **23.12.85 GB 8531624**

(43) Date of publication of application: **01.07.87 Bulletin 87/27**

(45) Publication of the grant of the patent: **11.04.90 Bulletin 90/15**

(84) Designated Contracting States: **BE CH DE ES FR GB IT LI NL**

(56) References cited:
**EP-A- 0 106 379**
**EP-A- 0 186 228**
**EP-A- 0 198 521**
**US-A- 4 414 409**

(73) Proprietor: **SHELL INTERNATIONALE RESEARCH MAATSCHAPPIJ B.V., Carel van Bylandtlaan 30, NL-2596 HR Den Haag(NL)**

(72) Inventor: **Drent, Eit, Badhuisweg 3, NL-1031 CM Amsterdam(NL)**

(74) Representative: **Aalbers, Onno et al, P.O. Box 302, NL-2501 CH The Hague(NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



ACTORUM AG

## Description

The invention relates to a process for the carbonylation of an ethylenically unsaturated compound in which each of the carbon atoms of the carbon-carbon double bond is secondary or tertiary.

European patent application 0, 106, 379 describes a process in which ethylenically unsaturated compounds are carbonylated with carbon monoxide in the presence of water, an alcohol and/or a carboxylic acid, a palladium catalyst, at least 5 mol of a triarylphosphine per gram atom of palladium and an acid with a $pK_a$ below 2, except hydrohalogenic acids and carboxylic acids.

This known process proceeds relatively slowly when ethylenically unsaturated compounds in which each of the carbon atoms of the carbon-carbon double bond is secondary or tertiary are used as starting compounds.

US-A-4, 414, 409 provides for a process for carbonylation of olefins using i.a. a 1,6 di(diphenylphosphino)hexane bidentate ligand, which process does not employ corrosive halides and which provides an increase in reaction rate in comparison with that achieved using known strong acids such as sulphuric acid. This process also proceeds relatively slowly when the ethylenically unsaturated olefins mentioned above are used.

It has now been found that the reaction rate can be very much enhanced by the presence of a bidentate ligand defined more closely hereinafter.

Accordingly, the invention provides a process for the carbonylation of an ethylenically unsaturated compound in which each of the carbon atoms of the carbon-carbon double bond is secondary or tertiary, with carbon monoxide in the presence of water and/or an alkanol, which process is carried out in the presence of a catalytic system prepared by combining:

a) palladium and/or a palladium compound,
b) an acid having a $pK_a$ below 2.0, measured at 18 °C in aqueous solution, except hydrohalogenic and carboxylic acids, and
c) a bidentate ligand having the general formula I

$$R^1R^2\text{-}M\text{-}R\text{-}M\text{-}R^3R^4 \quad (I)$$

wherein M represents a phosphorus, arsenic or antimony atom, R represents a divalent organic bridging group having in the range of from three to five carbon atoms in the bridge, none of these carbon atoms carrying substituents that may cause steric hindrance, and each of $R^1$, $R^2$, $R^3$ and $R^4$ represents an optionally substituted hydrocarbon group, and using a molar ratio of acid having a $pK_a$ below 2.0 to said bidentate ligand of greater than 0.5.

The acid having a $pK_a$ below 2.0 preferably has a non-coordinating anion, by which is meant that little or no covalent interaction takes place between the palladium and the anion (cf. GB-A 2,058,074). Typical examples of such anions are $PF_6^-$, $SbF_6^-$, $BF_4^-$ and $C10_4^-$.

Preferred acids are for example, sulphonic acids and acids that can be formed, possibly in situ, by interacting a Lewis acid such as, for example $BF_3$, $AsF_5$, $SbF_5$, $PF_5$, $TaF_5$ or $NbF_5$ with a Broensted acid such as, for example, a hydrohalogenic acid, in particular HF, fluorosulphonic acid, phosphoric acid or sulphuric acid. Specific examples of acids of the latter type are fluorosilicic acid, $HBF_4$, $HPF_6$ and $HSbF_6$. Examples of usable sulphonic acids are fluorosulphonic acid and chlorosulphonic acid and the hereinafter specified sulphonic acids.

A preferred group of acids having a $pK_a$ below 2.0 has the general formula II

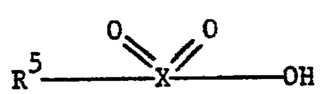

(II)

wherein X represents a sulphur or a chlorine atom and, if X represents a chlorine atom, $R^5$ represents an oxygen atom and, if X represents a sulphur atom, $R^5$ represents an OH group or an optionally substituted hydrocarbon group.

When the hereinbefore stated acids are used in the process according to the invention, the anions of the acids can be considered to be non-coordinating.

In the acids having the general formula I, the optionally substituted hydrocarbon group represented by $R^5$, is preferably an alkyl, aryl, aralkyl or alkaryl group having 1-30, in particular 1-14, carbon atoms. The hydrocarbon group may, for example, be substituted with the halogen atoms, in particular fluorine atoms.

Examples of suitable acids of the general formula II are perchloric acid, sulphuric acid, 2-hydroxypropane-2-sulphonic acid, p-toluenesulphonic acid and trifluoromethanesulphonic acid, the last two acids being the most preferred. The acid of the general formula II can also be an ion exchanger containing sulphonic acid groups, such as, for example, Amberlite 252 H ("Amberlite" is a trade name). In that case,

the hydrocarbon group $R^5$ represents a polymeric hydrocarbon group substituted with sulphonic acid groups, for example a polystyrene group.

Where, in the bidentate ligand, it is said that substituents offering steric hindrance should be absent, this means that no substituents may be present that are able to hinder the formation of complex compounds having the general formula III

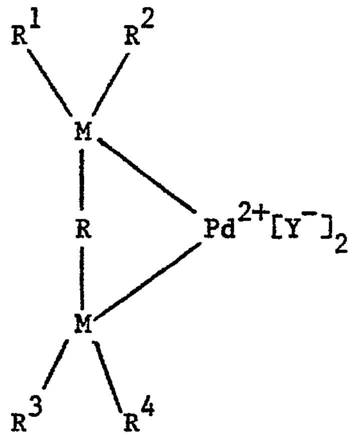

III

In formula III, Y represents a non-coordinating anion, whilst $Pd^{2+}$ can also be written as

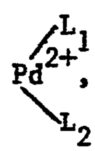

in which the ligands $L_1$ and $L_2$ are weakly coordinated solvent ligands, e.g. acetonitrile, methanol, acetone, or acetylacetone, or correspond with those employed in the palladium compounds described hereinafter.

In the bidentate ligand, M is preferably phosphorous. Hydrocarbon groups $R^1$, $R^2$, $R^3$ and $R^4$ will as a rule contain in the range of from 2 to 18 carbon atoms, preferably 6 to 14 carbon atoms. Aryl groups are the most suitable, in particular the phenyl group. The phenyl groups may be substituted, for example with halogen atoms and alkyl, aryl, alkoxy, carbalkoxy, trihalogen-methyl, cyano or dialkylamino groups. Preferred bridging groups -R- are those having the formula $-(-CR^6R^7-)_n-$ in which $R^6$ and $R^7$ each represent a hydrogen atom or an optionally substituted hydrocarbon group offering no steric hindrance and n is an integer in the range of from, 3 to 5. Substituents $R^6$ and $R^7$ preferably represent hydrogen atoms. The bridging groups R may also make part of a cyclic structure, e.g. an aromatic or cycloaliphatic group, the carbon to carbon bond or bonds in the bridge may be saturated or unsaturated and in the bridge or in the cyclic or non-cyclic groups attached to the bridge one or more hetero atoms, e.g. sulphur, oxygen, iron or nitrogen, may have been substituted for carbon atoms, other than the two carbon atoms which must be present in the bridge linking both atoms M.

Examples of suitable bidentate ligands are
1,3-di(diphenylphosphino)propane,
1,4-di(diphenylphosphino)butane,
2,3-dimethyl-1,4-di(diphenylphosphino)butane,
1,5-di(methyl-phenyl-phosphino)pentane,
1,4-di(dicyclohexylphospino)butane,
1,5-di(dinaphthylphosphino)pentane,
1,3-di(di-p-tolylphosphino)propane,
1,4-di(di-p-methoxyphenylphosphino)butane,
2,3-di(diphenylphosphino)-2-butene,
1,3-di(diphenylphosphino)-2-oxapropane,
2-methyl,2-(methyldiphenylphosphino)-1,3-di(diphenylphoshino)propane,
0,0′-di(diphenylphosphino)biphenyl,
1,8-di(diphenylphosphino)naphthalene and
bis(α,α′-diphenylphosphino)o-xylene.

The bidentate ligand can be used in quantities, relative to palladium and/or palladium compound, that can range within wide limits, suitably below 25, e.g. from 0.1 to 20 mol per mol palladium compound. Preferred quantities range from 0.3 to 10 mol per mol.

In addition to the bidentate ligand, one or more monodentate ligands can also be used in the preparation of the catalytic system. Suitable monodentate ligands are in particular optionally substituted triarylphosphines, such as triphenylphosphine, the phenyl groups optionally being substituted with alkyl, alkoxy, halogen and trihalomethyl groups. The use of an excess of monodentate ligand in relation to palladium and/or the palladium compound is recommended. Preferred quantities range from 2:1 to 50:1 mol in relation to palladium and/or palladium compound.

The carbon monoxide can be used in pure form or diluted with an inert gas such as hydrogen, nitrogen, noble gases or carbon dioxide in the process according to the invention.

The process of this invention is preferably carried out at a temperature between 20 and 225 °C, in particular between 50 and 200 °C, the total pressure ranges between preferably 1 and 100, in particular 20 and 75, bar gauge. It can be carried out batchwise, continuously or semi-continuously.

Both homogeneous and heterogeneous palladium compounds can be used. Homogeneous systems are preferred. Suitable palladium compounds are salts of palladium with, for example, nitric acid, sulphuric acid or alkanoic acids having not more than 12 carbon atoms per molecule. Salts of hydrohalogenic acids are theoretically also usable, but have the drawback that the halogen ion may have a corrosive action. Palladium carboxylates are the catalyst compounds preferably used, in particular palladium acetate. In addition, palladium acetylacetonate can also be used. Palladium on carbon and palladium bonded to an ion exchanger, for example one containing sulphonic acid groups, are examples of suitable heterogeneous palladium compounds.

The quantity of palladium and/or palladium compound is not critical. Preferably, quantities between $10^{-8}$ and $10^{-1}$ mol palladium and/or palladium compound per mol ethylenically unsaturated starting compound are used. The molar ratio of ethylenically unsaturated starting compound to carbon monoxide will as a rule range from 5:95 to 95:5, preferably from 1:5 to 5:1.

The process according to the present invention is carried out using a molar ratio of acid having a $pK_a$ below 2.0 to bidentate ligand of the general formula I of greater than 0.5, the rate of reaction being very low if this molar ratio is not greater than 0.5. Preferably, a molar ratio of acid to bidentate ligand in the range of from 1 to 10 is used; if desired, this molar ratio may be higher than 10, up to, for example, 25.

The process according to the present invention can be carried out conveniently in the presence of an aprotic solvent. A variety of solvents can be applied, such as ethers, for example diethyl ether, dimethyl ether of diethylene glycol (also referred to as "diglyme"), anisole and diphenyl ether; aromatic compounds, such as benzene, toluene, ethylbenzene and the three xylenes; halogenated aromatic compounds, such as chlorobenzene and the three dichlorobenzenes; halogenated alkanes, such as methylene chloride and carbontetrachloride; alkanes, such as hexane, heptane and 2,2,3-trimethylpentane; cycloalkanes, such as cyclohexane and methylcychlohexane; nitriles, such as benzonitrile and acetonitrile; esters, such as methyl benzoate, methyl acetate and dimethyl phthalate; sulphones, such as diethyl sulphone and tetrahydrothiophene 1,1-dioxide (also referred to as "sulpholane"). Also mixtures of solvents can be suitably applied. It is also possible to use an excess of the starting alkanol as solvent. Very good results have been obtained with aromatic hydrocarbons and with ethers.

The ethylenically unsaturated compound in which each of the carbon atoms of the carbon-carbon double bond is secondary, which is a carbon atom bound to two other carbon atoms, or tertiary, which is a carbon atom bound to three other carbon atoms, may be an alkene having up to 30 carbon atoms per molecule, such as 2-butene, 2-pentene, 2-hexene, 3-hexene, 2-heptene, 3-heptene, 2-octene, 3-octene, 4-octene, 2-nonene, 3-nonene, 4-nonene; a cycloalkene having up to 30 carbon atoms per molecule, such as cyclohexene, cycloheptene, cyclo-octene, cyclododecene and methylcyclo-octene; an alkyl or a phenyl ester of an alkenoic acid having up to 30 carbon atoms per molecule, such as crotonic acid, 2-pentenoic acid, 3-pentenoic acid, 2-hexenoic acid, 3-hexenoic acid, the alkyl group suitably having up to 30 carbon atoms.

Carbonylation in the presence of water leads to formation of carboxylic acids and carbonylation in the presence of alkanols to formation of carboxylic esters. The alkanol suitably has up to 30 carbon atoms per molecule and may be substituted with one or more substituents, for instance, with one or more halogen atoms or cyano, ester, alkoxy or aryl groups. Examples of suitable alkanols are methanol, ethanol, propanol, 2-propanol, 2-butanol, tert-butyl alcohol and chlorocapryl alcohol. Very good results have been obtained with methanol and ethanol.

The molar ratio of the ethylenically unsaturated compound to water and/or alkanol is not critical and may vary within wide limits, suitably between 0.1:1 and 10:1. When using a monoethylenically unsaturated compound, preference is usually given to the use of an excess of the alkanol.

The carboxylic acid or ester obtained by means of the present process may be isolated from the reaction mixture in any suitable manner, for example by means of distillation. Suitably, the acid or ester are obtained in a distillate fraction and an aprotic solvent is chosen which remains, together with the catalytic system, in the bottom fraction of the distillation. The bottom fraction may be re-used as catalytic system in the process of the present invention.

The following Examples further illustrate the invention.

Examples 1 and 2 and Comparative Experiments A-E

A 250-ml magnetically stirred Hastelloy C autoclave ("Hastelloy" is a trade mark) was charged with a mixture of n-octenes (20 ml), a solvent (30 ml), methanol (10 ml), palladium (II) acetate (0.4 mmol), ligand and acid. The table hereinafter states which solvent and which amounts of ligand and acid were used. The mixture of n-octenes consisted of 2.5% 1-octene, 39% 2-octene, 39% 3-octene and 19.5% 4-octene. The autoclave was flushed with carbon monoxide, filled with carbon monoxide at a pressure of 40 bar, sealed and heated to a temperature of 155 °C. After the reaction the contents of the autoclave were analysed by gas-liquid chromatography. The table states the conversion of n-octenes, the reaction rate in mol of n-octenes per mol of Pd per h and the content of unbranched ester. The symbol $\phi$ represents a phenyl group and pTsA refers to p-toluenesulphonic acid.

Table

| Example | Comparative Experiment | Ligand (mmol) | Acid (mmol) | Solvent | Conversion, % | Reaction rate | Unbranched ester, % |
|---|---|---|---|---|---|---|---|
| 1 | | $\phi_2P(CH_2)_3P\phi_2$ (1.6) | pTsA (4) | anisole | 80 | 130 | 69 |
| 2 | | $\phi_2P(CH_2)_4P\phi_2$ (1.6) | ditto | toluene | 93 | 155 | 69.3 |
| | A | $\phi_2P(CH_2)_2P\phi_2$ (1.6) | ditto | anisole | 0 | 0 | - |
| | B | $\phi_2P(CH_2)_6P\phi_2$ (1.6) | ditto | ditto | 38 | 30 | 64 |
| | C | $\phi_2P(CH_2)_4P\phi_2$ (1.6) | 2,6-dichlorobenzoic acid (20) | ditto | 47 | 40 | 59 |
| | D | ditto | $CF_3COOH$ (4) | ditto | 11 | 5 | 55 |
| | E | $P\phi_3$ (3.2) | pTsA (4) | toluene | 43 | 35 | 55 |

Comparative Experiment A shows that despite the use of an excess of acid with respect to the bidentate ligand $\phi_2P(CH_2)_2P\phi_2$ (not according to invention) no octene conversion had been observed.

Comparative Experiment B shows that despite the use of an excess of acid with respect to the bidentate ligand $\phi_2P(CH_2)_6P\phi_2$ (not according to invention) the rate at which the product is formed is relatively low.

Example 1 shows that when an excess of acid with respect to the bidentate ligand $\phi_2P(CH_2)_3P\phi_2$ is used, a large increase of the reaction rate is observed.

Example 2 shows that when an excess of acid with respect to the bidentate ligand $\phi_2P(CH_2)_4P\phi_2$ is used, a high reaction rate is obtained.

Comparative Experiments C and D show that when a carboxylic acid (not according to invention) having a $pK_a$ below 2.0 is used, the rate at which the product is formed is relatively low.

Comparative Experiment E shows that when triphenylphosphine (not according to invention) is used, the rate at which the product is formed is relatively low.

Example 3

In the way of Example 2 an experiment was carried out in which, instead of toluene (30 ml), diglyme (30 ml) and instead of methanol (10 ml), water (5 ml), was used. The conversion of n-octenes was 55%, the reaction rate was 110 mol of n-octenes per mol of Pd per h and the content of unbranched ester was 62%.

Example 4

In the way of Example 2 an experiment was carried out in which, instead of n-octenes (20 ml), ethyl crotonate (10 ml) was used.

The conversion of ethyl crotonate was 100%, the reaction rate was 180 mol of ethyl crotonate per mol of Pd per h and the content of unbranched diester was 31%, the diesters being a mixture of dimethyl glutarate, methyl ethyl glutarate and diethyl glutarate.

Example 5

In the way of Example 2 an experiment was carried out in which, instead of methanol (10 ml), ethanol (15 ml) and instead of n-octenes (20 ml), ethyl crotonate (10 ml) was used.

The conversion of ethyl crotonate was 100%, the reaction rate was 180 mol of ethyl crotonate per mol of Pd per h and the content of unbranched diester was 81.5%, the unbranched diester being diethyl glutarate.

Comparative Experiment F

In the way of Example 5 an experiment was carried out in which 10 instead of 1.6 mmol of $\phi_2P(CH_2)_4P\phi_2$ was used, the molar ratio of acid to ligand being 0.4.

The conversion of ethyl crotonate was less than 5% and the reaction rate was less than 5 mol of ethyl crotonate per mol of Pd per h.

Example 6

In the way of Example 5 an experiment was carried out in which 3.2 instead of 1.6 mmol of $\phi_2P(CH_2)_4P\phi_2$ was used, the molar ratio of acid to ligand being 1.25. The conversion of ethyl crotonate was 95%, the reaction rate was 100 mol of ethyl crotonate per mol of Pd per h and the content of unbranched diester was 69%, the unbranched diester being diethyl glutarate.

Example 7

In the way of Example 5 an experiment was carried out in which 40 instead of 15 ml of ethanol was used and no toluene was present.

The conversion of ethyl crotonate was 100%, the reaction rate was 150 mol of ethyl crotonate per mol of Pd per h and the content of unbranched diester was 83%, the unbranched diester being diethyl glutarate.

Example 8

In the way of Example 5 an experiment was carried out in which, instead of toluene (30 ml), anisole (30 ml) was used.

The conversion of ethyl crotonate was 100%, the reaction rate was 160 mol of ethyl crotonate per mol of Pd per h and the content of unbranched diester was 83%, the unbranched diester being diethyl glutarate.

Example 9

In the way of Example 8 an experiment was carried out in which ethyl crotonate (10 ml) was replaced with a mixture of cis and trans ethyl 3-pentenoate.

The conversion of ethyl 3-pentenoate was 100%, the reaction rate was 150 mol of ethyl 3-pentenoate per mol of Pd per h and the content of unbranched diester was 70%, the unbranched diester being diethyl adipate.

Comparative Experiment G

In the way of Example 9 an experiment was carried out in which $\phi_2P(CH_2)_4P\phi_2$ (1.6 mmol) was replaced with triphenylphosphine (3.2 mmol) and anisole (30 ml) with toluene (30 ml).

No conversion of ethyl crotonate had been observed.

Comparative Experiment H

In the way of Example 8 an experiment was carried out in which p-toluenesulphonic acid (4 mmol) was replaced with hydrogen chloride (4 mmol). No conversion of ethyl crotonate had been observed.

Example 10

In the way of Example 6 an experiment was carried out in which 0.8 instead of 3.2 mmol of $\phi_2P(CH_2)_4P\phi_2$ was used and also triphenylphosphine (2 mmol) was added.

The conversion of ethyl crotonate was 100%, the reaction rate was 120 mol of ethyl crotonate per mol of Pd per h and the content of unbranched diester was 82%, the unbranched diester being diethyl glutarate.

## Claims

1. A process for the carbonylation of an ethylenically unsaturated compound in which each of the carbon atoms of the carbon-carbon double bond is secondary or tertiary, with carbon monoxide in the presence of water and/or an alkanol, which process is carried out in the presence of a catalytic system prepared by combining:-

a) palladium and/or a palladium compound,

b) an acid having a $pK_a$ below 2.0, measured at 18 °C in aqueous solution, except hydrohalogenic and carboxylic acids, and

c) a bidentate ligand having the general formula I

$$R^1R^2\text{-}M\text{-}R\text{-}M\text{-}R^3R^4 \quad (I)$$

wherein M represents a phoshorous, arsenic or antimony atom, R represents a divalent organic bridging group having in the range of from three to five carbon atoms in the bridge, none of these carbon atoms carrying substituents that may cause steric hindrance, and each of $R^1$, $R^2$, $R^3$ and $R^4$ represents an optionally substituted hydrocarbon group,

and using a molar ratio of acid having a $pK_a$ below 2.0 to said bidentate ligand of greater than 0.5.

2. A process as claimed in claim 1, in which the acid having a $pK_a$ below 2.0 has a non-coordinating anion.

3. A process as claimed in claim 1 or 2, in which the acid having a $pK_a$ below 2.0 is a sulphonic acid or an acid that can be formed by interaction of a Lewis acid with a Broensted acid.

4. A process as claimed in claim 1 or 2, in which the acid having a $pK_a$ below 2.0 has the general formula II

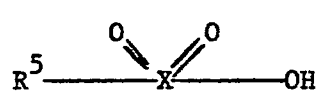

(II)

wherein X represents a sulphur or a chlorine atom and, if X represents a chlorine atom, $R^5$ represents an oxygen atom and, if X represents a sulphur atom, $R^5$ represents an OH group or an optionally substituted hydrocarbon group.

5. A process as claimed in claim 4, in which the optionally substituted hydrocarbon group represented by $R^5$ is an alkyl, aryl, aralkyl or alkaryl group having 1-30 carbon atoms.

6. A process as claimed in claim 4 or 5, in which the acid is p-toluenesulphonic acid or trifluoromethanesulphonic acid.

7. A process as claimed in any one of the preceding claims, in which the group -R- in the general formula I represents a group $-(CR^6R^7)-_n$ in which $R^6$ and $R^7$ represent hydrogen atoms or optionally substituted hydrocarbon groups offering no steric hindrance and n is an integer in the range of from 3 to 5.

8. A process as claimed in any one of the preceding claims, in which $R^1$, $R^2$, $R^3$ and $R^4$ in the general formula I each represent an aryl group with in the range of from 6 to 14 carbon atoms.

9. A process as claimed in claim 8, in which the aryl groups are phenyl groups.

10. A process as claimed in any one of the preceding claims, in which a molar ratio of acid having a $pK_a$ below 2.0 to said bidentate ligand below 25 is used.

11. A process as claimed in any one of the preceding claims, in which in the range of from 0.3 to 10 mol of the bidentate ligand having the general formula I per mol of palladium and/or palladium compound is used.

12. A process as claimed in any one of the preceding claims, in which the bidentate ligand is a phosphine.

13. A process as claimed in any one of the preceding claims, in which a molar ratio of acid having a $pK_a$ below 2.0 to said bidentate ligand in the range of from 1 to 10 is used.

14. A process as claimed in any one of the preceding claims, which is carried out in the presence of a solvent comprising an aromatic hydrocarbon or an ether.

15. A process as claimed in any one of the preceding claims, in which the reaction mixture obtained is separated by means of distillation into a distillate fraction containing the carbonylation product and a residual fraction containing the catalytic system, which catalytic system is recycled to the process for reuse.

**Patentansprüche**

1. Verfahren zur Carbonylierung einer ethylenisch ungesättigten Verbindung, in der jedes der Kohlenstoffatome der Kohlenstoff-Kohlenstoff-Doppelbindung sekundär oder tertiär ist, mit Kohlenmonoxid in Gegenwart von Wasser und/oder einem Alkanol, wobei das Verfahren durchgeführt wird in Gegenwart eines Katalysatorsystems, das erhalten worden ist durch Zusammenbringen von
a) Palladium und/oder einer Palladiumverbindung,
b) einer Säure mit einem $pK_a$-Wert < 2,0, gemessen bei 18°C in wäßriger Lösung, mit Ausnahme von Halogenwasserstoff- und Carbonsäuren, und
c) einem zweibindigen Liganden der allgemeinen Formel I

$$R^1R^2\text{-}M\text{-}R\text{-}M\text{-}R^3R^4 \quad (I),$$

in der M ein Phosphor-, Arsen- oder Antimonatom bedeutet, R eine zweiwertige organische Brückengruppe mit drei bis vier Kohlenstoffatomen in der Brücke bedeutet, wobei keines dieser Kohlenstoffatome Substituenten enthält, die eine sterische Hinderung hervorrufen könnten, und jeder der Reste $R^1$, $R^2$, $R^3$ und $R^4$ eine gegebenenfalls substituierte Kohlenwasserstoffgruppe bedeutet, unter Anwendung eines Molverhältnisses von Säure mit einem $pK_a$-Wert < 2,0 zu dem zweibindigen Liganden > 0,5.

2. Verfahren nach Anspruch 1, wobei die Säure mit einem $pK_a$-Wert 2,0 ein nicht-koordinierendes Anion besitzt.

3. Verfahren nach Anspruch 1 oder 2, wobei die Säure mit einem $pK_a$-Wert < 2,0 eine Sulfonsäure oder eine Säure ist, die gebildet werden kann durch Wechselwirkung einer Lewis-Säure mit einer Broensted-Säure.

4. Verfahren nach Anspruch 1 oder 2, wobei die Säure mit einem $pK_a$-Wert 2,0 die allgemeine Formel II besitzt

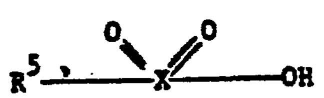 (II)

in der X ein Schwefel oder ein Chloratom bedeutet und, wenn X ein Chloratom ist, $R^5$ ein Sauerstoffatom bedeutet und, wenn X ein Schwefelatom ist, $R^5$ eine OH-Gruppe oder eine gegebenenfalls substituierte Kohlenwasserstoffgruppe bedeutet.

5. Verfahren nach Anspruch 4, wobei die gegebenenfalls substituierte Kohlenwasserstoffgruppe, die durch $R^5$ angegeben ist, eine Alkyl-, Aryl-, Aralkyl- oder Alkarylgruppe mit 1-30 Kohlenstoffatomen ist.

6. Verfahren nach Anspruch 4 oder 5, wobei die Säure p-Toluolsulfonsäure oder Trifluormethansulfonsäure ist.

7. Verfahren nach einem der vorangehenden Ansprüche, wobei die Gruppe -R- in der allgemeinen Formel I eine Gruppe

$$-(-CR^6R^7-)_n-$$

bedeutet, in der $R^6$ und $R^7$ Wasserstoffatome oder gegebenenfalls substituierte Kohlenwasserstoffgruppen, die keine sterische Hinderung hervorrufen, und n eine ganze Zahl im Bereich von 3 bis 5 bedeuten.

8. Verfahren nach einem der vorangehenden Ansprüche, wobei $R^1$, $R^2$, $R^3$ und $R^4$ in der allgemeinen Formel I jeweils eine Arylgruppe mit 6 bis 14 Kohlenstoffatomen bedeuten.

9. Verfahren nach Anspruch 8, wobei die Arylgruppen Phenylgruppen sind.

10. Verfahren nach einem der vorangehenden Ansprüche, wobei ein Molverhältnis von Säure mit einem $pK_a$-Wert$< 2{,}0$ zu zweibindigem Liganden $< 25$ angewandt wird.

11. Verfahren nach einem der vorangehenden Ansprüche, wobei 0,3 bis 10 mol zweibindiger Ligand der allgemeinen Formel I pro mol Palladium und/oder Palladiumverbindung angewandt werden.

12. Verfahren nach einem der vorangehenden Ansprüche, wobei der zweibindige Ligand ein Phosphin ist.

13. Verfahren nach einem der vorangehenden Ansprüche, wobei ein Molverhältnis von Säure mit einem $pK_a$-Wert $< 2{,}0$ zu zweibindigem Liganden im Bereich von 1 bis 10 angewandt wird.

14. Verfahren nach einem der vorangehenden Ansprüche, das durchgeführt wird in Gegenwart eines Lösungsmittels, umfassend einen aromatischen Kohlenwasserstoff oder einen Ether.

15. Verfahren nach einem der vorangehenden Ansprüche, bei dem das erhaltene Reaktionsgemisch durch Destillation aufgetrennt wird in eine Destillatfraktion, enthaltend das Carbonylierungsprodukt, und eine Rückstandsfraktion, enthaltend das katalytische System, wobei das katalytische System zur Wiederverwendung in das Verfahren zurückgeführt wird.

**Revendications**

1. Un procédé pour la carbonylation d'un composé éthyléniquement insaturé dans lequel chacun des atomes de carbone de la double liaison carbone-carbone est secondaire ou tertiaire, avec de l'oxyde de carbone en présence d'eau et/ou d'un alcanol, lequel procédé est mis en œuvre en présence d'un système catalytique préparé en combinant:

a) du palladium et/ou un composé du palladium,

b) un acide ayant un $pK_a$ au-dessous de 2,0, mesuré à 18°C en solution aqueuse, à l'exception des acides halogénhydriques et carboxyliques, et

c) un ligand bidenté ayant la formule générale I

$$R^1R^2\text{-}M\text{-}R\text{-}M\text{-}R^3R^4 \quad (I)$$

où M représente un atome de phosphore, d'arsenic ou d'antimoine, R représente un groupe organique divalent formant pont ayant de trois à cinq atomes de carbone dans le pont, aucun de ces atomes de carbone ne portant de substituants pouvant causer un empêchement stérique, et chacun de $R^1$, $R^2$, $R^3$ et $R^4$ représente un groupe d'hydrocarbure éventuellement substitué,

et en utilisant un rapport molaire de l'acide ayant un $pK_a$ au-dessous de 2,0 au ligand bidenté supérieur à 0,5.

2. Un procédé selon la revendication 1, dans lequel l'acide ayant un $pK_a$ au-dessous de 2,0 a un anion non-coordinateur.

3. Un procédé selon la revendication 1 ou 2, dans lequel l'acide ayant un $pK_a$ au-dessous de 2,0 est un acide sulfonique ou un acide qui peut être formé par interaction d'un acide de Lewis avec un acide de Broensted.

4. Un procédé selon la revendication 1 ou 2, dans lequel l'acide ayant un $pK_a$ au-dessous de 2,0 a la formule générale II

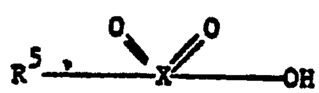

(II)

où X représente un atome de soufre ou de chlore et, si X représente un atome de chlore, $R^5$ représente un atome d'oxygène et, si X représente un atome de soufre, $R^5$ représente un groupe OH ou un groupe d'hydrocarbure éventuellement substitué.

5. Un procédé selon la revendication 4, dans lequel le groupe d'hydrocarbure éventuellement substitué représenté par $R^5$ est un groupe alcoyle, aryle, aralcoyle ou alcaryle ayant 1–30 atomes de carbone.

6. Un procédé selon la revendication 4 ou 5, dans lequel l'acide est de l'acide p-toluènesulfonique ou de l'acide trifluorométhanesulfonique.

7. Un procédé selon l'une quelconque des revendications précédentes, dans lequel le groupe -R- dans la formule générale I représente un groupe $-(-CR^6R^7-)-_n$ ou $R^6$ et $R^7$ représentent des atomes d'hydrogène ou des groupes d'hydrocarbures éventuellement substitués ne causant pas d'empêchement stérique et n est un nombre entier de 3 à 5.

8. Un procédé selon l'une quelconque des revendications précédentes, dans lequel $R^1$, $R^2$, $R^3$ et $R^4$ dans la formule générale I représentent chacun un groupe aryle ayant de 6 à 14 atomes de carbone.

9. Un procédé selon la revendication 8, dans lequel les groupes aryle sont des groupes phényle.

10. Un procédé selon l'une quelconque des revendications précédentes, dans lequel on utilise un rapport de l'acide ayant un $pK_a$ au-dessous de 2,0 au ligand bidenté au-dessous de 25.

11. Un procédé selon l'une quelconque des revendications précédentes, dans lequel on utilise de 0,3 à 10 moles du ligand bidenté ayant la formule générale I par mole de palladium et/ou de composé du palladium.

12. Un procédé selon l'une quelconque des revendications précédentes, dans lequel le ligand bidenté est une phosphine.

13. Un procédé selon l'une quelconque des revendications précédentes, dans lequel on utilise un rapport molaire de l'acide ayant un $pK_a$ au-dessous de 2,0 au ligand bidenté compris entre 1 et 10.

14. Un procédé selon l'une quelconque des revendications précédentes, qui est mis en œuvre en présence d'un solvant comprenant un hydrocarbure aromatique ou un éther.

15. Un procédé selon l'une quelconque des revendications précédentes, dans lequel le mélange de réaction obtenu est séparé par distillation en une fraction distillée contenant le produit de carbonylation et une fraction résiduelle contenant le système catalytique, lequel système catalytique est recyclé au procédé pour réutilisation.